(19) **Europäisches Patentamt / European Patent Office / Office européen des brevets**

(11) **EP 2 356 452 B1**

(12) # EUROPEAN PATENT SPECIFICATION

(45) Date of publication and mention of the grant of the patent:
**24.04.2019 Bulletin 2019/17**

(51) Int Cl.:
*G01N 33/50* (2006.01)     *G01N 33/74* (2006.01)

(21) Application number: **09744979.7**

(22) Date of filing: **29.10.2009**

(86) International application number:
**PCT/EP2009/007924**

(87) International publication number:
**WO 2010/049180 (06.05.2010 Gazette 2010/18)**

(54) **METHODS AND ASSAYS FOR CLASSIFYING FOODSTUFF AND/OR BEVERAGE AND/OR DIET AND/OR NUTRITION REGIMEN AND/OR MEDICAMENT IN VIEW OF AN EFFECT ON THE CARDIOVASCULAR SYSTEM**

VERFAHREN UND ASSAYS ZUR KLASSIFIZIERUNG VON LEBENSMITTELN UND/ODER GETRÄNKEN UND/ODER DIÄTEN UND/ODER ERNÄHRUNGSPLÄNEN UND/ODER MEDIKAMENTEN IN HINSICHT AUF EINEN EFFEKT AUF DAS HERZ-KREISLAUFSYSTEM

Procédés et analyses pour classifier aliments et/ou boissons et/ou régimes et/ou régimes nutritionnels et/ou médicaments susceptibles d'avoir un effet sur le système cardiovasculaire

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **31.10.2008 EP 08168107**

(43) Date of publication of application:
**17.08.2011 Bulletin 2011/33**

(73) Proprietor: **B.R.A.H.M.S GmbH**
**16761 Hennigsdorf (DE)**

(72) Inventors:
• **BERGMANN, Andreas**
**12351 Berlin (DE)**
• **PFEIFFER, Andreas F. H.**
**14558 Nuthetal (DE)**
• **RUDOVICH, Natalia**
**14558 Nuthetal (DE)**

(74) Representative: **Hertin und Partner Rechts- und Patentanwälte PartG mbB**
**Kurfürstendamm 54/55**
**10707 Berlin (DE)**

(56) References cited:
**DE-A1-102006 052 916     US-A1- 2008 213 746**

• **NERI S. ET AL.**: "Effects of antioxidant supplementation on postprandial oxidative stress and endothelial dysfunction: A single-blind, 15-day clinical trial in patients with untreated type 2 diabetes, subjects with impaired glucose tolerance, and healthy controls" CLIN. THERAP., vol. 27, no. 11, November 2005 (2005-11), pages 1764-1773, XP005215815
• **SUZUKI K. ET AL.**: "Improved early-phase insulin response after Candesartan treatment in hypertensive patients with impaired glucose tolerance" CLIN. EXP. HYPERTENSION, vol. 30, no. 5, July 2008 (2008-07), pages 309-314, XP002510787
• **MORGENTHALER N.G. ET AL.**: "Immunoluminometric assay for the midregion of pro-atrial natriuretic peptide in human plasma" CLIN. CHEM., vol. 50, no. 1, January 2004 (2004-01), pages 234-236, XP002510788 cited in the application
• **MORGENTHALER N.G. ET AL.**: "Measurement of midregional proadrenomedullin in plasma with an immunoluminometric assay" CLIN. CHEM., vol. 51, no. 10, October 2005 (2005-10), pages 1823-1829, XP002510789 cited in the application
• **MORGENTHALER N.G. ET AL.**: "Assay for the measurement of copeptin, a stable peptide derived from the precursor of vasopressin" CLIN. CHEM., vol. 52, no. 1, January 2006 (2006-01), pages 112-119, XP002510790 cited in the application

- MORGENTHALER N.G. ET AL.: "Copeptin: clinical use of a new biomarker" TRENDS ENDOCRINOL. METABOL., vol. 19, no. 2, March 2008 (2008-03), pages 43-49, XP002510791
- PAPASSOTIRIOU J. ET AL.: "Immunoluminometric assay for measurement of the C-terminal endothelin-I precursor fragment in human plasma" CLIN. CHEM., vol. 52, no. 6, June 2006 (2006-06), pages 1144-1151, XP002510792 cited in the application
- YOUSUFUDDIN M. ET AL.: "Incremental importance of peak-exercise plasma levels of endothelin-I and natriuretic peptides in chronic heart failure" J. CARDIOVASC. PHARMACOL., vol. 38, no. 3, September 2001 (2001-09), pages 468-473, XP009110790

**Description**

Subject of the invention is an *in vitro*-method for classifying a foodstuff and/or beverage and/or diet and/or nutrition regimen and/or medicament in view of an effect on the cardiovascular system of a subject, comprising determining the relative level of one or more cardiovascular markers as well as uses thereof.

Hypertension frequently accompanies obesity, hyperinsulinism and insulin resistance. This type of hypertension is characterized by sodium retention, an increased intravascular volume and increased cardiac stroke volume and output (Messerli FH, et al. 1981 Obesity and essential hypertension. Hemodynamics, intravascular volume, sodium excretion, and plasma renin activity. Arch Intern Med 141:81-5; Stelfox HT, et al. 2006 Hemodynamic monitoring in obese patients: the impact of body mass index on cardiac output and stroke volume. Crit Care Med 34:1243-6). Atrial natriuretic peptide (ANP) and Brain type natriuretic peptide (BNP) are synthesized in myocardial cells as a response to increased wall stress in relation to heart failure or acute myocardial ischemia as prohormones that are cleaved into ANP and BNP and N-terminal proANP (NT-proANP) as well as N-terminal proBNP (NT-proBNP), respectively (Ruskoaho H 2003 Cardiac hormones as diagnostic tools in heart failure. Endocr Rev 24:341-56; Potter LR, et al. 2006 Natriuretic peptides, their receptors, and cyclic guanosine monophosphate-dependent signaling functions. Endocr Rev 27:47-72). High levels of natriuretic peptides are new promising cardiovascular (CV) risk markers and have been associated with high blood pressure (BP), left ventricular hypertrophy, and albuminuria (Olsen MH, et al. 2005 N-terminal pro brain natriuretic peptide is inversely related to metabolic cardiovascular risk factors and the metabolic syndrome. Hypertension 46:660-6). Several large studies indeed observed independent associations of mortality and natriuretic peptide levels (Wang TJ, et al. 2004 Plasma natriuretic peptide levels and the risk of cardiovascular events and death. N Engl J Med 350:655-63; Bibbins-Domingo K, et al. 2007 N-terminal fragment of the prohormone brain-type natriuretic peptide (NT-proBNP), cardiovascular events, and mortality in patients with stable coronary heart disease. Jama 297:169-76; Kistorp C, et al. 2005 N-terminal pro-brain natriuretic peptide, C-reactive protein, and urinary albumin levels as predictors of mortality and cardiovascular events in older adults. Jama 293:1609-16; von Haehling, et al. 2007 Comparison of midregional pro-atrial natriuretic peptide with N-terminal pro-B-type natriuretic peptide in predicting survival in patients with chronic heart failure. J Am Coll Cardiol 50:1973-80).

Recent studies have described that natriuretic peptide levels are suppressed in obesity (Wang TJ, et al. 2004 Impact of obesity on plasma natriuretic peptide levels. Circulation 109:594-600; Das SR, et al. 2005 Impact of body mass and body composition on circulating levels of natriuretic peptides: results from the Dallas Heart Study. Circulation 112:2163-8). Since obesity is associated with salt retention and increased cardiac output it would be expected to produce elevated natriuretic peptide levels. That obesity seemed to have the opposite effect appeared counterintuitive and was attributed to nonhemodynamic factors. Wang and colleagues therefore postulated that this inverse relationship may be due to increased expression of the natriuretic peptide clearance receptor (NPR-C) by adipose tissue resulting in increased clearance of natriuretic peptides in obese subjects (Wang TJ, et al. 2004 Impact of obesity on plasma natriuretic peptide levels. Circulation 109:594-600). However, Das and colleagues determined lean and fat mass by DEXA in the Dallas heart study and observed an association of lower BNP levels with lean rather than fat mass (Das SR, et al. 2005 Impact of body mass and body composition on circulating levels of natriuretic peptides: results from the Dallas Heart Study. Circulation 112:2163-8).

Several studies observed an association of natriuretic peptides with further components of the metabolic syndrome (Olsen MH, et al. 2008 Cardiovascular risk prediction by N-terminal pro brain natriuretic peptide and high sensitivity C-reactive protein is affected by age and sex. J Hypertens 26:26-34; Wang TJ, et al. 2007 Association of plasma natriuretic peptide levels with metabolic risk factors in ambulatory individuals. Circulation 115:1345-53). Elevated waist circumference, elevated triglycerides, reduced HDL, and elevated fasting glucose (Wang TJ, et al. 2007 Association of plasma natriuretic peptide levels with metabolic risk factors in ambulatory individuals. Circulation 115:1345-53) were associated with lower plasma ANP levels, and somewhat less with lower BNP levels in the Framingham heart study. In a Danish study, an association of BNP with BMI, insulin, glucose, triglycerides and hypertension was observed (Olsen MH, et al. 2005 N-terminal pro brain natriuretic peptide is inversely related to metabolic cardiovascular risk factors and the metabolic syndrome. Hypertension 46:660-6). Although these studies demonstrated close links of the natriuretic peptides to several traits of the metabolic syndrome, the mechanisms behind these associations have remained elusive.

Nutritional approaches reducing postprandial insulin levels are highly effective in reducing blood pressure (Appel LJ, et al. 2005 Effects of protein, monounsaturated fat, and carbohydrate intake on blood pressure and serum lipids: results of the OmniHeart randomized trial. Jama 294:2455-64) and the postprandial state is believed to play an important role in the early stages of the metabolic syndrome and particularly in the development of atherosclerosis (Hanefeld M, et al. 1999 Postprandial plasma glucose is an independent risk factor for increased carotid intima-media thickness in non-diabetic individuals. Atherosclerosis 144:229-35; Hanefeld M, et al. 2007 The challenge of the Metabolic Syndrome. Horm Metab Res 39:625-6).

Suzuki K. et al., 2008, Clin. Exp. Hypertension 30(5): 309-314 discloses a method for determining early-phase insulin response after Candesartan treatment in hypertensive patients. Before and after administration, a 75 g oral glucose tolerance test (OGTT) was conducted to compare various parameters such as blood pressure, glucose cholesterol and triglycerides.

Object of the present invention was the classification of a foodstuff and/or beverage and/or diet and/or nutrition regimen and/or medicament in view of an effect on the cardiovascular system of a subject.

Surprisingly, it was found that insulin induces suppression of ANP. This connection provides a direct link from insulin resistance to hypertension in the metabolic syndrome. The finding of this connection further lead to methods for classifying a foodstuff and/or beverage and/or diet and/or nutrition regimen and/or medicament in view of an effect on the cardiovascular system of a subject and uses thereof.

Thus, subject of the present invention is an *in vitro*-method for classifying a foodstuff and/or beverage and/or diet and/or nutrition regimen and/or medicament in view of an effect on the cardiovascular system of a subject, comprising determining the relative level of one or more cardiovascular markers in said subject, wherein one of the cardiovascular markers is MR-proANP.

According to the present invention "relative level" is defined as being the relative concentration based on a basal value, which can be mathematically expressed as follows:

$$X = 100 \times \frac{[postprandial]}{[basal]}$$

wherein X is the change of the level of one or more cardiovascular markers in percent.

It can also be specified as percentaged change of the concentration relative to the basal value, which can be mathematically expressed as follows:

$$X = 100 - \frac{[postprandial]}{[basal]} * 100$$

or

$$X = \frac{[postprandial]}{[basal]} * 100 - 100$$

In a preferred embodiment of the inventive method the postprandial relative level of one or more cardiovascular markers is determined.

In the context of the present invention the term "postprandial" refers to the period of time after the foodstuff and/or beverage and/or medicament is ingested by or applied otherwise to the subject, which may also be in the context of a diet and/or nutrition regimen.

In the context of the present invention cardiovascular marker means a peptide and/or protein providing diagnosis and/or prognosis and/or monitoring of cardiovascular diseases (e.g. myocardial infarction, coronary artery disease, heart failure) selected from the group of natriuretic peptides (e.g. atrial natriuretic peptide, brain natriuretic peptide), adrenomedullin, endothelins, vasopressin. The basal level of the cardiovascular markers depends on such factors as the subject's age, body mass index, genetic predisposition for certain conditions/ family history, gender and ethnic background of the patient, as well as on the overall health status of said subject. The present invention, however, is based on the finding that in contrast to this, the relative change from the basal level of the cardiovascular markers to the postprandial level of the cardiovascular markers is essentially independent from these factors and strongly depends on foodstuff and/or beverage and/or diet and/or nutrition regimen and/or medicament which is administered to the subject.

In an especially preferred embodiment of the inventive method the relative level of one or more cardiovascular markers is determined with an assay having a lower detection limit of 1 nmol/L or lower, preferably 100 pmol/L or lower, more preferably 10 pmol/L or lower, even more preferably 1 pmol/L or lower, most preferably 0.5 pmol/L or lower. Furthermore, the assay preferably has an interassay precision of < 30%, more preferably < 20% in the normal range. Furthermore, the assay has an intraassay precision of < 5% in the measuring range. Hereby "intraassay precision" specifies the deviance between measurements within a single batch of a specific assay, and "interassay precision" specifies the deviance between measurements within multiple batches of a specific assay, which may be carried out in different locations, on different days, by different operators. Thus, the aforementioned terms are related to a measure of the reproducibility of results obtained with the concerned assays. "Measuring range" specifies the upper and lower limit of detection of an assay.

The assay is at least sensitive enough to detect changes and variances as increase and as decrease. For a healthy subject the normal range of a given biomarker corresponds to a Gaussian distribution.

An embodiment of the invention is further an *in vitro*-method according to the present invention, further comprising:

a) determining the basal level of one or more cardiovascular markers in said subject,
b) determining the postprandial level of said one or more cardiovascular markers,
c) calculating the relative level of one or more cardiovascular markers from the values obtained in steps a and b.

Hereby, the ingestion, intake or other form of application of said foodstuff and/or beverage and/or diet and/or nutrition regimen and/or medicament is correlated to its influence on the level of said one or more cardiovascular markers in said subject in terms of a relative decrease or increase of said level.

In the context of the present invention, the term "basal level" refers to the individual level of a certain compound, molecule or metabolite, such as a cardiovascular peptide, which a subject has without the influence of factors such as a foodstuff, a beverage, a diet, a nutrition regimen or a medicament. Said basal level is individually determined for each subject after approximately 12 hours of fasting. Fasting hereby means that the subject does not ingest or otherwise consume foodstuffs, beverages or medicaments for a certain amount of time, except water and/or indispensable medication.

In a preferred embodiment of the invention a postprandial relative increase of the level of said one or more cardiovascular markers of more than 5%, preferably between more than 5 and up to 20% in said subject is correlated with a positive effect of said foodstuff and/or beverage and/or diet and/or nutrition regimen and/or medicament on the cardiovascular system of said subject, wherein a postprandial relative increase or decrease of the level of said one or more cardiovascular markers of about 5% in said subject is correlated to a neutral effect of said foodstuff and/or beverage and/or diet and/or nutrition regimen and/or medicament on the cardiovascular system of said subject, and wherein a postprandial relative decrease of the level of said one or more cardiovascular markers of more than 5% in said subject is correlated to a negative effect of said foodstuff and/or beverage and/or diet and/or nutrition regimen and/or medicament on the cardiovascular system of said subject.

A positive effect on the cardiovascular system is, in a medical sense, the improvement of the cardiovascular system of said subject, or, if the subject is healthy, the preservation of the medical condition of said subject.

A neutral effect on the cardiovascular system is, in a medical sense, the preservation of the medical condition of the cardiovascular system of said subject.

A negative effect on the cardiovascular system is, in a medical sense, the pejoration of the medical condition of the cardiovascular system of said subject.

A positive effect on the cardiovascular system of said subject may include cardiovascular remodelling and improvement of cardiovascular function, which may especially and preferably include the prevention of atherosclerosis, hypertension and cardiac and vascular dysfunction and development of heart failure.

In another preferred embodiment the basal level of said cardiovascular marker and the postprandial level of said cardiovascular marker in said subject are determined with an immunoassay.

The diagnostic assay can be of any type applied in the field of diagnostics, including but not restricted to assay methods based on enzymatic reactions, luminescence, fluorescence, or radiochemicals. The preferred detection methods comprise strip tests, radioimmunoassay, chemiluminescence- and fluorescence-immunoassay, Immunoblot assay, Enzyme-linked immunoassay (ELISA), Luminex-based bead arrays, and protein microarray assay. The assay types can further be microtiter plate-based, chip-based, bead-based, wherein the biomarker proteins can be attached to the surface or are in solution. The assays can be homogenous or heterogeneous assays, sandwich assays, competitive and non-competive assays (The Immunoassay Handbook, Ed. David Wild, Elsevier LTD, Oxford; 3rd ed. (May 2005), ISBN-13: 978-0080445267; Hultschig C et al., Curr Opin Chem Biol. 2006 Feb;10(1):4-10. PMID: 16376134).

In the most preferred embodiment of the invention an immunoassay is used as described in (Morgenthaler NG et al; 2004 ClinChem 50:234-6).

In the *in vitro*-method according to the invention one of the cardiovascular markers is midregional proANP. Mostly preferred is midregional proANP$_{53-90}$.

In another preferred embodiment of the *in vitro*-method according to the invention the postprandial level of said one or more cardiovascular markers is determined within 4 hours, preferably within 2 hours, more preferably between 15 and 60 minutes after administration of said foodstuff and/or beverage and/or diet and/or nutrition regimen.

In another preferred embodiment of the *in vitro*-method according to the invention the postprandial influence of said foodstuff and/or beverage and/or diet and/or nutrition regimen on the relative level of one or more cardiovascular peptides is monitored over a prolonged period, preferred over a period of one week, more preferred one month, even more preferred two months, even more preferred half a year.

Subject of the invention is further the use of an assay, preferably having a sensitivity of 1 nmol/L or lower, preferably 100 pmol/L or lower, more preferably 10 pmol/L or lower, even more preferably 1 pmol/L or lower, most preferably 0.5 pmol/L or lower for the methods of the present invention.

In one aspect of the method according to the invention the change of the level of one or more cardiovascular markers of a subject relative to the basal level of said markers of said subject is determined, wherein the change is a decrease.

Subject of the invention is further the use of an assay for determining the change of the level of one or more cardiovascular markers of a subject relative to the basal level of said markers of said subject, wherein the assay is capable of detecting a decrease of the level of said one or more cardiovascular markers and capable of detecting an increase of the level of said one or more cardiovascular markers.

It is important to note that the capability of the assay used in the present invention to measure a decrease in the level of said one or more cardiovascular markers is critical, in particular in healthy subjects, wherein the decrease leads to very low levels of said one or more cardiovascular markers. Thus, the assay used herein preferably is ultra-sensitive in order to be capable of measuring a decrease in the level of said one or more cardiovascular markers, in subjects in which the basal level lies within the 97.5$^{th}$ percentile of said level in the healthy population.

Subject of the invention is further the use of an assay as described above, wherein the change is an increase or a decrease, and wherein the assay has sensitivity of 1 nmol/L or lower.

Subject of the invention is further the use of an assay as described above, for determining the postprandial change of the level of one or more cardiovascular markers of a subject relative to the basal level of said markers of said subject, wherein one of the cardiovascular markers is MR-proANP.

Subject of the invention is further the use of an assay having a sensitivity of 1 nmol/L or lower, preferably 100 pmol/L or lower, more preferably 10 pmol/L or lower, even more preferably 1 pmol/L or lower, most preferably 0.5 pmol/L or lower for determining the change of the level of one or more cardiovascular markers of a subject relative to the basal level of said markers of said subject, wherein the change is an increase or a decrease. In the method the postprandial relative level of one or more cardiovascular markers is determined, wherein one of the cardiovascular markers is midregional proANP. Mostly preferred is midregional proANP$_{53-90}$.

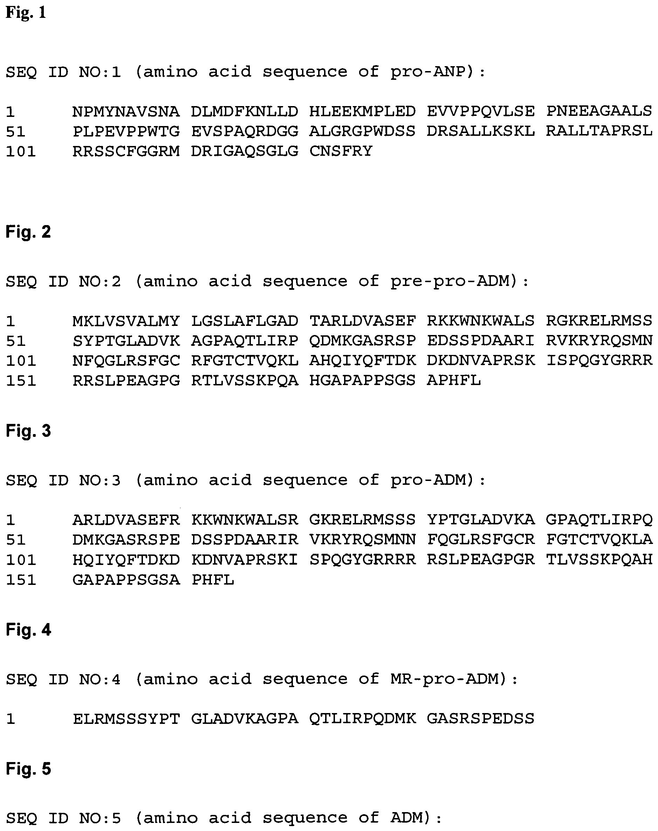

MR-proANP$_{53-90}$ specifies the midregional pro-atrial natriuretic peptide, which comprises amino acids 53 to 90 of the pro-atrial natriuretic peptide (proANP), Figure 1.

Subject of the invention is further the use of an assay, preferably having a sensitivity of 1 nmol/L or lower, preferably 100 pmol/L or lower, more preferably 10 pmol/L or lower, even more preferably 1 pmol/L or lower, most preferably 0.5 pmol/L or lower, for determining the postprandial change of the level of one or more cardiovascular markers of a subject relative to the basal level of said markers of said subject, wherein the change is an increase or a decrease.

It is preferred that the assay is an immunoassay.

Subject of the invention is further the use of an assay as outlined above, wherein said change in the level of one or more cardiovascular markers is used to classify a foodstuff and/or beverage and/or diet and/or nutrition regimen and/or medicament in view of an effect on the cardiovascular system of a subject.

In a particular embodiment said change in the level of one or more cardiovascular markers is used to classify a diabetes medicament.

In one embodiment of the invention said subject is a healthy or non-symptomatic human being.

In another embodiment of the invention said subject is a human being having a medical condition associated with the cardiovascular system and/or metabolic syndrome.

As used herein, the term "metabolic syndrome" refers to the aggregation of several risk factors for cardiovascular diseases and type II diabetes, as defined by the American Heart Association (AHA) and National Heart, Lung and Blood Institute (NHLBI) (Grundy et al. 2005. Circulation 112: 2735-3752). Important components of the metabolic syndrome are, among others, glucose intolerance and dyslipidemias, hypertension and central obesity. The metabolic syndrome is diagnosed if 3 of the following 5 criteria are fulfilled: elevated waist circumference ($\geq$ 102 cm in male and $\geq$ 88 cm in female), elevated triglycerides ($\geq$ 150 mg/dL/ 1.7 mmol/L, respectively, or drug treatment for elevated triglycerides), reduced HDL-cholesterol (< 40 mg/dL/ 1.03 mmol/L, respectively, in male; < 50 mg/dL/ 1.3 mmol/L, respectively, in female; or drug treatment for reduced HDL-cholesterol), elevated blood pressure ($\geq$ 130 mm Hg systolic blood pressure or $\geq$ 85 mm Hg diastolic blood pressure or on antihypertensive drug treatment in a subject with a history of hypertension) and elevated fasting glucose ($\geq$ 100 mg/dL or drug treatment for elevated glucose).

A more recent definition with some modifications has been given by the International Diabetes Federation (http://www.idf.org/webdata/docs/IDF_Meta_def_final.pdf).

In one embodiment of the invention said subject is a human being suffering from diabetes, in particular type II diabetes.

In a preferred embodiment of the invention, the condition associated with the cardiovascular system and/or metabolic system is the metabolic syndrome.

A condition associated with the cardiovascular system and/or metabolic system may also comprise a state after incidents of the cardiovascular system and/or metabolic system. In these cases healthy nutrition may improve the subject's chance of survival (de Lorgeril M, et al. Mediterranean diet, traditional risk factors, and the rate of cardiovascular complications after myocardial infarction: final report of the Lyon Diet Heart Study. Circulation 1999; 99(6): 779-85).

In another preferred embodiment of the invention, the condition associated with the cardiovascular system and/or metabolic system is selected from the group comprising myocardial infarction (MI), coronary syndromes, congestive heart failure (CHF), coronary artery disease (atherosclerosis), stroke, transient ischemic attacks (TIA), periphery artery disease, cardiomyopathy, diabetes mellitus type II, renal failure and/or subjects with one or more symptoms of the above mentioned diseases, e.g. obesity, hypertension, headache, chest pain and dyspnea.

According to the present disclosure it is preferred that at least one of the markers is selected from the group comprising natriuretic peptides, endothelin-1 (ET-1), vasopressin (AVP), adrenomedullin (ADM), as well as propeptides thereof and fragments of at least 3 amino acids thereof, preferably more than 5, more preferably more than 6, even more preferably more than 7, even more preferably more than 10, even more preferably more than 12, even more preferably more than 15, most preferably 20 or more.

Natriuretic peptide refers to a peptide which induces natriuresis (the discharge of sodium through urine). Types include:

- Artrial natriuretic peptide,
- Brain natriuretic peptide,
- C-type natriuretic peptide.

According to the present disclosure said marker is a natriuretic peptide or a propeptide or fragments of at least 3 amino acids thereof, preferably more than 5, more preferably more than 6, most preferably more than 7.

At least one of the cardiovascular markers is atrial natriuretic peptide (ANP) or a propeptide or fragments of at least 3 amino acids thereof, preferably more than 5, more preferably more than 6, most preferably more than 7.

At least one of the cardiovascular markers is MR-proANP$_{53-90}$ or fragments of at least 3 amino acids thereof, preferably more than 5, more preferably more than 6, most preferably more than 7.

AVP in the context of the present disclosure relates to arginine vasopressin (= vasopressin) or fragments thereof or precursors or fragments thereof. A preferred fragment of a precursor of AVP is C-terminal proAVP (CT-proAVP or Copeptin). CT-proAVP$_{107-145}$ (or CT-pre-proAVP$_{126-164}$) is a particularly preferred marker peptide in the context of the present invention.

ADM in the context of the present disclosure relates to adrenomedullin or fragments thereof or precursors or fragments thereof. A preferred fragment of a precursor of ADM is midregional proADM (MR-proADM). MR-proADM$_{24-71}$ (or MR-preproADM$_{45-92}$) is a particularly preferred marker peptide.

ET-1 in the context of the present disclosure relates to endothelin 1 or fragments thereof or precursors or fragments thereof. A preferred fragment of a precursor of ET-1 is C-terminal-proETI (CT-proET1). CT-proET-1$_{151-195}$ (or CT-preproET-1$_{168-212}$) is a particularly preferred marker peptide.

**Figure Description**

Figure 1
Sequence of pro-ANP

Figure 2
Sequence of pre-pro-ADM

Figure 3
Sequence of pro-ADM

Figure 4
Sequence of MR-pro-ADM

Figure 5
Sequence of ADM

Figure 6
Sequence of pre-pro-ET-1

Figure 7

Sequence of pro-ET-1

Figure 8
Sequence of ET-1

Figure 9
Sequence of CT-pro-ET-1

Figure 10
Sequence of Big-ET-1

Figure 11
Sequence of pre-pro-AVP

Figure 12
Sequence of pro-AVP

Figure 13
Sequence of AVP

Figure 14
Sequence of CT-pre-proAVP (Copeptin)

Figure 15
Plasma MR-proANP$_{53-90}$ (A) and serum insulin (B) during the oral glucose tolerance test in non obese normotensive subjects (black diamonds), normotensive subjects with central obesity (white diamonds) and hypertensive subjects (black triangles); * $p < 0.05$ non obese vs. obese, normotensive subjects; † $p < 0.001$ obese normotensive vs. hypertensive subjects; ‡ $p < 0.01$ hypertensive vs. non obese normotensive subjects.
(C) Supression of plasma MR-proANP$_{53-90}$ levels in the hyperinsulinemic, euglycemic clamps. (D) Delta MR-proANP$_{0-120\ min}$ in subjects with low and high insulin sensitivity, determined as glucose infusion rate (GIR) values in the steady-state of the clamp below the 25$^{th}$ and above the 75$^{th}$ percentile, respectively. Data are shown by box-and-whiskers-plots. The box extends from the 25$^{th}$ to the 75$^{th}$ percentile, with a line at the median indicating the 50$^{th}$ percentile. The whiskers represent the ranges extending from the lowest to the highest value.

Figure 16
Comparison of relative concentrations of NT-proBNP and MR-proANP$_{53-90}$ after oGTT in n=10 subjects.

## Examples

RESEARCH DESIGN AND METHODS

**Study protocol**

The study protocol was approved by the ethical committees of the Potsdam University and Charite University of Medicine, Berlin, Germany. Before the study, informed written consent was obtained from all participants.

**Study design**

The subjects are part of an ongoing case-control association study of the aetiology of the metabolic syndrome and type 2 diabetes mellitus (Metabolic Syndrome Berlin-Potsdam Study, MESY-BEPO). In Potsdam and Berlin, Germany, volunteers from the general population were recruited. The baseline examination included anthropometric measurements, blood sampling, a 75 g oral glucose tolerance test (oGTT) and personal interview on lifestyle habits and medical history. A subgroup of this population (n = 31) underwent hyperinsulinemic, euglycemic clamps, which was conducted on a separate day after the oGTT.

**Subjects**

One hundred and eight non hypertensive subjects (55 non-obese and 53 with central obesity) and 54 patients with an essential hypertension were studied. Hypertension was defined as systolic blood pressure $\geq$140 mm Hg, diastolic blood pressure $\geq$90 mm Hg, or use of antihypertensive therapy. All drug treated hypertensive subjects had a stable medication in the last six month prior the study. Subjects with elevations in liver enzymes more than twice the respective upper normal limits, or with elevated serum creatinine concentrations (> 1.3 mg/dl) or with severe conditions including generalized inflammation, heart failure or end-stage malignant diseases were excluded from the study. All subjects were instructed to maintain their normal physical activity and to consume a normal diet containing $\geq$200 g of carbohydrate during three days before oGTT and clamp test. Subjects with antidiabetic therapy or newly diagnosed type 2 diabetes mellitus were excluded from the examination. Definitions of disturbances in the glucose metabolism were based on the 1997 American Diabetes Association criteria for glucose values obtained after an overnight fast and a two-hour 75 g oGTT (2000 Report of the Expert Committee on the Diagnosis and Classification of Diabetes Mellitus. Diabetes Care 23 Suppl 1:S4-19).

### Experimental procedures

All tests were performed in the morning after 12 hours overnight fast. BP was measured by a trained study nurse using an Omron® HEM705CP manometer (Omron, Germany) with patients in the sitting position. Three measurements were taken at 2-min intervals and the average was used to define clinical systolic and diastolic blood pressures. For oGTT venous blood samples were drawn at 0, 30, 60, 90, 120 and 180 min relative to the oral glucose loading. Euglycemic, hyperinsulinemic clamp:

Hyperinsulinemic euglycemic clamps were performed for 120 min using 100 mU of human insulin per $m^2$ of the body surface per min (Actrapid; Novo Nordisk, Bagsværd, Denmark) and a variable infusion of 20% glucose (Serag Wiessner, Naila, Germany) (DeFronzo RA, et al. 1979 Glucose clamp technique: a method for quantifying insulin secretion and resistance. Am J Physiol 237:E214-23). In the steady-state condition of the clamp, capillary blood glucose was adjusted at 5.5 mmol/l for at least 60 min. A deviation of a single capillary glucose concentration of > 10% during assumed steady-state conditions was defined as non-steady state. Throughout the clamp, capillary blood glucose concentrations were monitored every 5 min and used to regulate plasma glucose by the adjustment of a variable infusion of glucose.

### Analytical procedures

All venous blood samples were immediately centrifuged and frozen at - 70°C until analyzed. Capillary blood glucose concentrations were determined using a glucose oxidase method on Dr. Müller G-L (Dr. Müller Glucose analyzer, Freital, Germany). Serum triglycerides, total cholesterol and HDL-cholesterol were determined by standard enzymatic assays, and LDL-cholesterol calculated from these data (certified laboratory for clinical chemistry). HbAlc was determined using a Hi-Auto A1C HA-8140 system (Menarini Diagnostics, Germany). Serum insulin was measured using a commercial enzyme-linked immunosorbent assay (Insulin ELISA, Mercodia AB, Uppsala, Sweden). Homeostasis Model Assessment Insulin Resistance (HOMA-IR) was calculated as fasting insulin (IU/L) x fasting glucose (mmol/L) / 22.5 (Matthews DR, et al. 1985 Homeostasis model assessment: insulin resistance and beta-cell function from fasting plasma glucose and insulin concentrations in man. Diabetologia 28:412-9).

Human plasma MR-proANP$_{53-90}$ was determined as described previously (Morgenthaler NG, et al. 2004 Immunoluminometric assay for the midregion of pro-atrial natriuretic peptide in human plasma. Clin Chem 50:234-6).

NT-proBNP was determined by an electrochemiluminescence immunoassay (ELICIA, Roche Diagnostics, Basel, Switzerland).

### Statistical analysis

We divided the study population into three groups: patients with essential hypertension (n = 54), non hypertensive subjects with central obesity (n = 53), and non hypertensive subjects without central obesity (n = 55). Central obesity was diagnosed according to the ATP III-defined metabolic syndrome criteria (National Cholesterol Education Program (NCEP) Expert Panel on Detection, Evaluation, and Treatment of High Blood Cholesterol in Adults (Adult Treatment Panel III). Third report of the national Cholesterol Education Program (NCEP) expert panel on detection, evaluation, and treatment of high blood cholesterol in adults (Adult Treatment Panel III). Final report. Circulation 2002; 106:3143-3421).

General characteristics are given as mean $\pm$ SD. All other data are presented as means $\pm$ SEM. All data were log-transformed before analysis. Delta MR-proANP$_{53-90}$ from 0 to 180 min was calculated in oGTT. Group comparison was performed by ANOVA followed by the Sidak test as post hoc multiple group comparison. Repeated measures ANOVA analyses were used for comparison of the time-courses of MR-proANP$_{53-90}$ during oGTT between three groups. Correlation analysis was made by Pearson's correlation. In clamp experiments, insulin sensitivity was determined as glucose infusion rate (GIR; mg per kg body weight $\cdot$ min$^{-1}$) in the steady-state of the clamp test (least 30 min) divided by circulating insulin concentration in steady-state (pmol/l). The nonparametric Wilcoxon's signed-rank test for paired samples was used to compare data from the baseline and in the steady-state. P values < 0.05 were considered significant in all analyses. All statistical analyses were performed using SPSS for Windows 14 (SPSS Inc., Chicago, Illinois).

RESULTS

The characteristics of the study population are summarised in Table 1. Obese normotensive subjects were more insulin resistant compared with non obese normotensive subjects: they had higher fasting insulin and blood glucose concentrations, lower HDL-cholesterol concentration and triglyceride levels, and higher SPB and DBP. Except higher age, HbAlc level and SBP, obese subjects with hypertension were comparable in BMI, waist circumference and insulin resistance with obese normotensive subjects.

The lowest fasting concentrations of MR-proANP$_{53-90}$ were observed in obese normotensive subjects, compared with non obese normotensive subjects and obese subjects with hypertension (53.9 $\pm$ 28.0 pmol/l vs. 64.1 $\pm$ 25.6 pmol/l and 77.5 $\pm$ 30.8 pmol/1). After adjustment for age and BMI, the differences remained significant. Fasting MR-proANP$_{53-90}$ levels correlated significantly and positively with age (r = 0.429, p < 0.0001), HDL-cholesterol (r = 0.270, p = 0.006), and negatively with BMI (r = -0.313, p = 0.001), DBP (r = -0.251, p = 0.009), fasting insulin (r = -0.276, p = 0.004) and HOMA-IR index (r = -0.268, p = 0.005) in normotensive subjects. By contrast, in obese subjects with hypertension positive correlations with MR-proANP$_{53-90}$ were restricted to age (r = 0.546, p<0.0001).

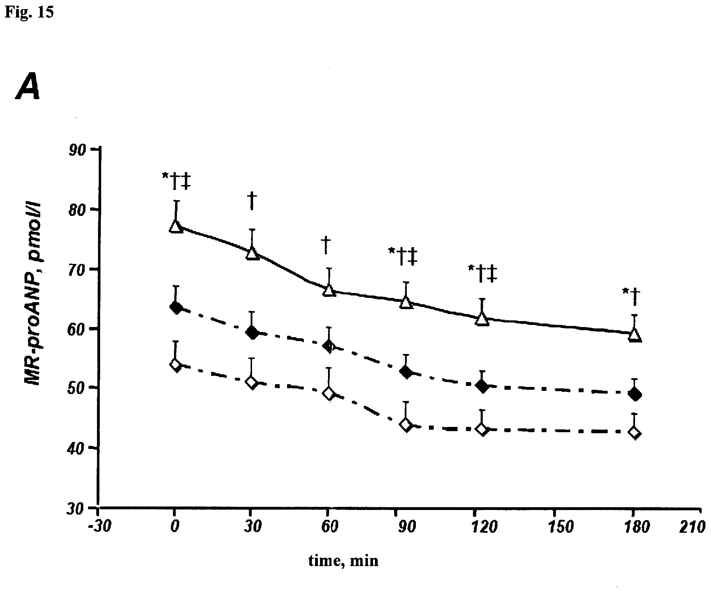

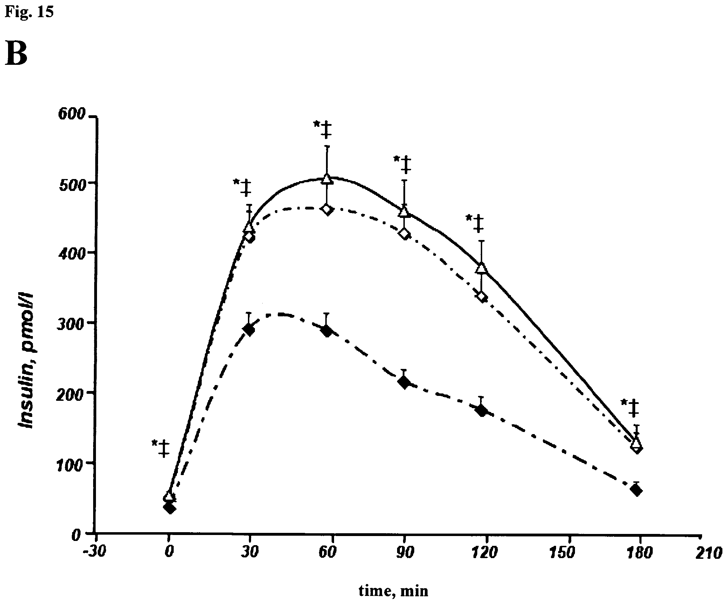

In all study subjects, MR-proANP$_{53-90}$ levels decreased rapidly at 30 min after oral glucose challenge and remained suppressed during the entire test (p < 0.0001; for basal levels vs. levels at 30, 60, 90, 120 and 180 min of the oGTT). Post challenge concentrations of MR-proANP$_{53-90}$ were significantly lower (Fig. 15A) from 90 to 180 min in obese normotensive subjects compared with non obese normotensive subjects and obese hypertensive subjects. The relative suppression of MR-proANP$_{53-90}$ at 180 min was 20.0 $\pm$ 13.4 % in non obese normotensive subjects vs. 21.4 $\pm$ 19.5 % in obese normotensive subjects vs. 21.2 $\pm$ 13.4% in hypertensive subjects (not significant). Fasting and post glucose challenge levels of insulin were significantly lower in obese normotensive subjects (Fig. 15B) and correlated negatively with fasting and post challenge levels of MR-proANP$_{53-90}$ in normotensive subjects from 60 to 180 min (r = -0.198 - -0.358; p < 0.0001 - 0.05), while these correlations were much weaker in hypertensive subjects. There was no correlation of fasting and post challenge blood glucose levels with MR-proANP$_{53-90}$ levels during oGTT in normotensive and hypertensive subjects (data not shown).

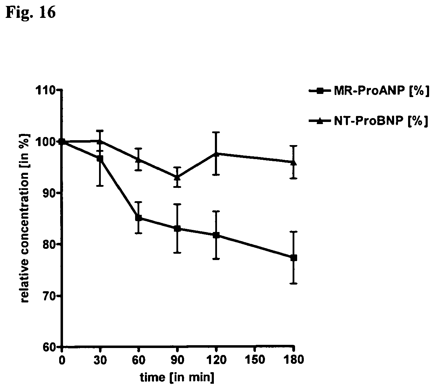

Although BNP appears to play a minor role physiologically in healthy subjects, it is usually regulated in a similar manner to ANP. We therefore tested whether NT-proBNP levels also decline in response to oral glucose challenges. NT-proBNP levels were assessed in 10 subjects and compared to MR-proANP$_{53-90}$ in this subgroup. Indeed, the relative concentrations of NT-proBNP also declined after the glucose challenge, but the response was more transient and smaller compared to MR-proANP$_{53-90}$ (Fig. 16).

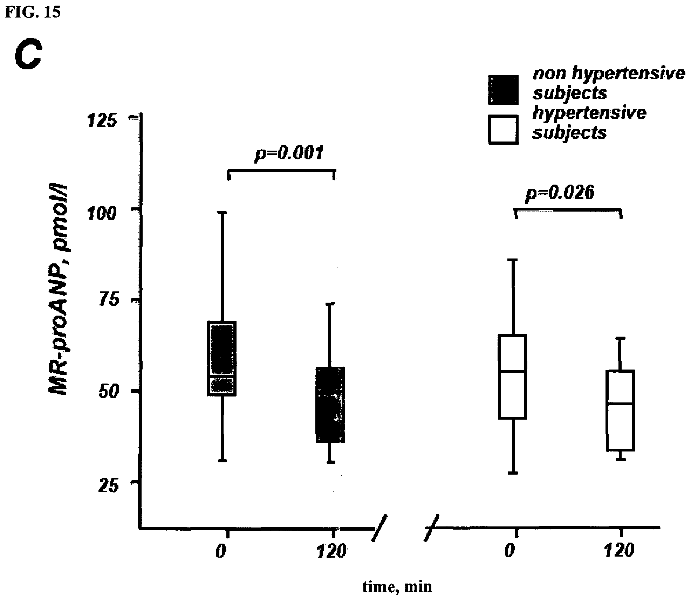

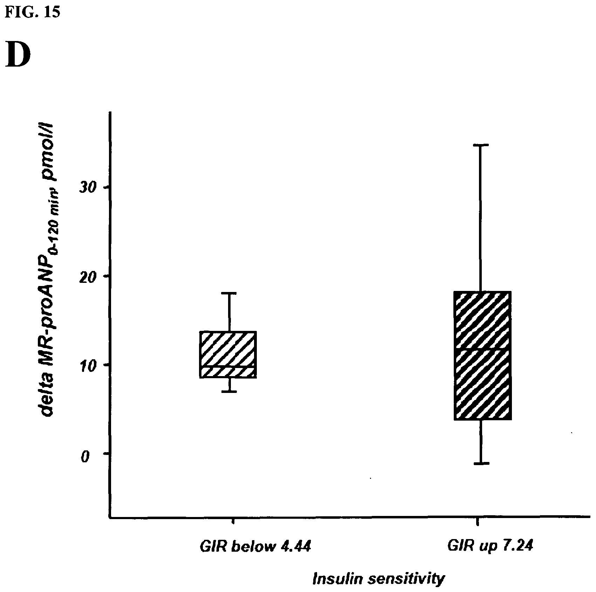

Thirty-one obese individuals (17 normotensive subjects and 14 hypertensive subjects) underwent euglycemic hyperinsulinemic clamps. Normotensive subjects were matched for age, BMI, waist circumference and insulin sensitivity with hypertensive subjects (mean $\pm$ SE; 30.6 $\pm$ 3.5 kg/m2 vs. 32.0 $\pm$ 3.4 kg/m2, p = 0.341; 101.0 $\pm$ 7.3 cm vs. 103.6 $\pm$ 8.8 cm, p = 0.526; 6.1 $\pm$ 1.6 mg/kg body weight x min-1 vs. 5.5 $\pm$ 2.0 mg/kg body weight x min-1, p = 0.388; respectively). Fasting blood glucose, insulin and MR-proANP$_{53-90}$ levels were not different between normotensive and hypertensive subjects (mean $\pm$ SE; 5.2 $\pm$ 0.5 mmol/l vs. 5.4 $\pm$ 0.6 nimol/l; p = 0.147; 66.0 $\pm$ 40.2 pmol/l vs. 76.2 $\pm$ 39.0 pmol/l; p = 0.470; 59.5 $\pm$ 18.3 pmol/l vs. 61.0 $\pm$ 31.5 pmol/l; p = 0.874; respectively). In the euglycemic clamp, circulating insulin levels increased to 1223 (range 708 - 2106) pmol/l in normotensive subjects and to 1247 (range 430 - 1476) pmol/l in hypertensive subjects at 120 min of the clamp (p = 0.489). In both groups MR-proANP$_{53-90}$ levels were significantly decreased at 120 min of the clamp compared with basal values, but they did not differ between both groups ($\Delta$ MR-proANP$_{53-90}$ 0-120 min 11.1 (-1.3 - 25.1) pmol/l in normotensive subjects vs. 8.5 (-35.2 - 34.6) pmol/l in hypertensive subjects; p = 0.297) (Fig. 15C). No difference was observed in the suppression of MR-proANP$_{53-90}$ in subjects with low and high insulin sensitivity (determined as glucose infusion rate value below the 25$^{th}$ and above the 75$^{th}$ percentile, respectively) (Fig. 15D).

Mean MR-ProADM concentration in healthy individuals (n=264) was 0.33 nmol/L (standard deviation 0.07 nmol/L), range 0.1 - 0.64 nmol/L, 99$^{th}$ percentile was 0.52 nmol/L, 97.5$^{th}$ percentile was 0.49 nmol/L, 2.5$^{th}$ percentile was 0.17 nmol/L, 1$^{st}$ percentile was 0.14 nmol/L. The lower detection limit of the assay was 0.08 nmol/L (Morgenthaler et al. 2005. Clin Chem 51(10):1823-1829).

Median MR-ProANP$_{53-90}$ concentration in healthy individuals (n=325) was 45 pmol/L, range 9.6 - 313 pmol/L, 99$^{th}$ percentile was 197.5 pmol/L, 97.5$^{th}$ percentile was 163.9 pmol/L, 2.5$^{th}$ percentile was 18.4 pmol/L, 1$^{st}$ percentile was 13.6 pmol/L. The lower detection limit of the assay was 6.0 pmol/L (Morgenthaler et al. 2004. Clin Chem 50(1):234-236).

Median CT-ProAVP concentration in healthy individuals (n=359) was 4.2 pmol/L, range 1 - 13.8 pmol/L, 99$^{th}$ percentile was 13.5 pmol/L, 97.5$^{th}$ percentile was 11.25 pmol/L, 2.5$^{th}$ percentile was 1.7 pmol/L. The lower detection limit of the assay was 1.7 pmol/L (Morgenthaler et al. 2006. Clin Chem 52(1):112-119). 9 individuals out of 359 had CT-proAVP-values below the lower detection limit and were defined as 1.0 pmol/L.

Mean CT-ProET-1 concentration in healthy individuals (n=326) was 44.3 pmol/L (standard deviation 10.6 pmol/L), range 10.5 - 77.4 pmol/L, 99th percentile was 72.8 pmol/L, 97.5th percentile was 66.6 pmol/L, 2.5th percentile was 24.8 pmol/L, 1st percentile was 17.4 pmol/L. The lower detection limit of the assay was 0.4 pmol/L (Papassotiriou et al. 2006. Clin Chem 52(6):1144-1151).

Mean NT-proBNP concentration in healthy individuals (n=2264) was 5.94 pmol/l (standard deviation 7.36 pmol/l), the median was 3.25 pmol/l, 97.5th percentile was 19.94 pmol/l and 95th percentile was 17.58 pmol/l. The lower detection limit of the assay was 0.59 pmol/l (Assay proBNP II cobas by Roche; manual 2007-09 V2).

**Table 1** Clinical and biochemical characteristics of the study subjects

| | Non obese, non hypertensive subjects (n= 55) | Obese, non hypertensive subjects (n= 53) | Obese, hypertensive subjects (n= 54) |
|---|---|---|---|
| **Clinical characteristics** | | | |
| Age (years) | 47.1 ± 13.7 | 47.9 ± 10.5 | 60.1 ± 8.8[a, d] |
| Sex (female; %) | 74.2 | 73.4 | 66.2 |
| BMI ($kg/m^2$) | 23.4 ± 2.3 | 30.0 ± 5.0[a] | 30.0 ± 4.0[a] |
| Waist circumference (cm) | 79.8 ± 8,7 | 100.0 ± 10.4[a] | 100.1 ± 11.4[a] |
| Systolic blood pressure (mm Hg) | 111.3 ± 12.1 | 119.5 ± 13.6[a] | 130.9 ± 16.8[a, d] |
| Diastolic blood pressure (mm Hg) | 70.6 ± 8.2 | 78.5 ± 8.3[a] | 81.6 ± 10.9[a] |
| **Biochemical characteristics** | | | |
| Fasting blood glucose (mmol/l) | 4.7 ± 0.4 | 5.0 ± 0.5[b] | 5.0 ± 0.6[b] |
| HbAlc (%) | 5.3 ± 0.4 | 5.3 ± 0.4 | 5.6 ± 0.4[a, d] |
| HDL cholesterol (mmol/1) | 1.5 ± 0.3 | 1,.3 ± 0.3[a] | 1.3 ± 0.3[a] |
| Triglycerides (mmol/l) | 1.0 ± 0.6 | 1.6 ± 0.9[b] | 1.6 ± 0.8[a] |
| Fasting insulin (pmol/l) | 36.8 ± 47.0 | 52.9 ± 34.9[a] | 54.1 ± 35.8[a] |
| $HOMA_{IR}$ (mU mmol/l) | 1.3 ± 1.9 | 2.0 ± 1.4[a] | 2.0 ± 1.5[a] |
| proANP (pmol/l) 0 min delta $proANP_{(0-180 min)}$ | 64.1 ± 25.6 | 53.9 ± 28.0[c] | 77.5 ± 30.8[d] |
| (pmol/l) | 14.7 ± 11.9 | 10.9 ± 11.0[c] | 18.2 ± 15.3[d] |

Unless otherwise indicated, values are means ± SD. All values are unadjusted. [a]p < 0.0001; [b]p < 0.01; [c]p<0.05 vs. non obese, non hypertensive subjects and [d]p<0.0001; [e]p<0.05 vs. obese non hypertensive subjects. Obesity was defined as "central obesity" by ATIII Criteria for metabolic syndrome: waist circumference for women > 88 cm and > 102 cm for men.

SEQUENCE LISTING

<110> B.R.A.H.M.S. AG

<120> Methods and Assays for classifying Foodstuff and/or Beverage and/or Diet and/or Nutrition Regimen and/or Medicament in view of an Effect on the cardiovascular system

<130> B60414PCT

<150> EP08168107.4
<151> 2008-10-31

<160> 14

<170> PatentIn version 3.3

<210> 1
<211> 126
<212> PRT
<213> Homo sapiens

<400> 1

```
Asn Pro Met Tyr Asn Ala Val Ser Asn Ala Asp Leu Met Asp Phe Lys
1               5                   10                  15

Asn Leu Leu Asp His Leu Glu Glu Lys Met Pro Leu Glu Asp Glu Val
            20                  25                  30

Val Pro Pro Gln Val Leu Ser Glu Pro Asn Glu Glu Ala Gly Ala Ala
        35                  40                  45

Leu Ser Pro Leu Pro Glu Val Pro Pro Trp Thr Gly Glu Val Ser Pro
    50                  55                  60

Ala Gln Arg Asp Gly Gly Ala Leu Gly Arg Gly Pro Trp Asp Ser Ser
65                  70                  75                  80

Asp Arg Ser Ala Leu Leu Lys Ser Lys Leu Arg Ala Leu Leu Thr Ala
                85                  90                  95

Pro Arg Ser Leu Arg Arg Ser Ser Cys Phe Gly Gly Arg Met Asp Arg
            100                 105                 110

Ile Gly Ala Gln Ser Gly Leu Gly Cys Asn Ser Phe Arg Tyr
        115                 120                 125
```

<210> 2
<211> 185
<212> PRT
<213> Homo sapiens

<400> 2

```
Met Lys Leu Val Ser Val Ala Leu Met Tyr Leu Gly Ser Leu Ala Phe
1               5                   10                  15

Leu Gly Ala Asp Thr Ala Arg Leu Asp Val Ala Ser Glu Phe Arg Lys
```

```
                    20                      25                      30

      Lys Trp Asn Lys Trp Ala Leu Ser Arg Gly Lys Arg Glu Leu Arg Met
              35              40                  45

      Ser Ser Ser Tyr Pro Thr Gly Leu Ala Asp Val Lys Ala Gly Pro Ala
          50              55                  60

      Gln Thr Leu Ile Arg Pro Gln Asp Met Lys Gly Ala Ser Arg Ser Pro
      65              70                  75                      80

      Glu Asp Ser Ser Pro Asp Ala Ala Arg Ile Arg Val Lys Arg Tyr Arg
                      85                  90                      95

      Gln Ser Met Asn Asn Phe Gln Gly Leu Arg Ser Phe Gly Cys Arg Phe
                  100                 105                 110

      Gly Thr Cys Thr Val Gln Lys Leu Ala His Gln Ile Tyr Gln Phe Thr
                  115             120                 125

      Asp Lys Asp Lys Asp Asn Val Ala Pro Arg Ser Lys Ile Ser Pro Gln
          130             135                 140

      Gly Tyr Gly Arg Arg Arg Arg Ser Leu Pro Glu Ala Gly Pro Gly
      145             150                 155                 160

      Arg Thr Leu Val Ser Ser Lys Pro Gln Ala His Gly Ala Pro Ala Pro
                  165                 170                 175

      Pro Ser Gly Ser Ala Pro His Phe Leu
                  180             185
```

<210> 3
<211> 164
<212> PRT
<213> Homo sapiens

<400> 3

```
      Ala Arg Leu Asp Val Ala Ser Glu Phe Arg Lys Lys Trp Asn Lys Trp
      1               5                   10                  15

      Ala Leu Ser Arg Gly Lys Arg Glu Leu Arg Met Ser Ser Ser Tyr Pro
                  20                  25                  30

      Thr Gly Leu Ala Asp Val Lys Ala Gly Pro Ala Gln Thr Leu Ile Arg
                  35              40                  45

      Pro Gln Asp Met Lys Gly Ala Ser Arg Ser Pro Glu Asp Ser Ser Pro
          50                  55                  60

      Asp Ala Ala Arg Ile Arg Val Lys Arg Tyr Arg Gln Ser Met Asn Asn
      65                  70                  75                  80
```

```
            Phe Gln Gly Leu Arg Ser Phe Gly Cys Arg Phe Gly Thr Cys Thr Val
                            85              90              95

            Gln Lys Leu Ala His Gln Ile Tyr Gln Phe Thr Asp Lys Asp Lys Asp
                        100             105             110

            Asn Val Ala Pro Arg Ser Lys Ile Ser Pro Gln Gly Tyr Gly Arg Arg
                        115             120             125

            Arg Arg Arg Ser Leu Pro Glu Ala Gly Pro Gly Arg Thr Leu Val Ser
                    130             135             140

            Ser Lys Pro Gln Ala His Gly Ala Pro Ala Pro Pro Ser Gly Ser Ala
            145             150             155             160

            Pro His Phe Leu
```

<210> 4
<211> 40
<212> PRT
<213> Homo sapiens

<400> 4

```
            Glu Leu Arg Met Ser Ser Ser Tyr Pro Thr Gly Leu Ala Asp Val Lys
            1               5               10              15

            Ala Gly Pro Ala Gln Thr Leu Ile Arg Pro Gln Asp Met Lys Gly Ala
                        20              25              30

            Ser Arg Ser Pro Glu Asp Ser Ser
                    35              40
```

<210> 5
<211> 52
<212> PRT
<213> Homo sapiens

<400> 5

```
            Tyr Arg Gln Ser Met Asn Asn Phe Gln Gly Leu Arg Ser Phe Gly Cys
            1               5               10              15

            Arg Phe Gly Thr Cys Thr Val Gln Lys Leu Ala His Gln Ile Tyr Gln
                        20              25              30

            Phe Thr Asp Lys Asp Lys Asp Asn Val Ala Pro Arg Ser Lys Ile Ser
                        35              40              45

            Pro Gln Gly Tyr
                    50
```

<210> 6
<211> 212
```

<212> PRT
<213> Homo sapiens

<400> 6

```
        Met Asp Tyr Leu Leu Met Ile Phe Ser Leu Leu Phe Val Ala Cys Gln
        1               5                   10                  15

        Gly Ala Pro Glu Thr Ala Val Leu Gly Ala Glu Leu Ser Ala Val Gly
                    20                  25                  30

        Glu Asn Gly Gly Glu Lys Pro Thr Pro Ser Pro Pro Trp Arg Leu Arg
                    35                  40                  45

        Arg Ser Lys Arg Cys Ser Cys Ser Ser Leu Met Asp Lys Glu Cys Val
            50                  55                  60

        Tyr Phe Cys His Leu Asp Ile Ile Trp Val Asn Thr Pro Glu His Val
        65                  70                  75                  80

        Val Pro Tyr Gly Leu Gly Ser Pro Arg Ser Lys Arg Ala Leu Glu Asn
                        85                  90                  95

        Leu Leu Pro Thr Lys Ala Thr Asp Arg Glu Asn Arg Cys Gln Cys Ala
                    100                 105                 110

        Ser Gln Lys Asp Lys Lys Cys Trp Asn Phe Cys Gln Ala Gly Lys Glu
                    115                 120                 125

        Leu Arg Ala Glu Asp Ile Met Glu Lys Asp Trp Asn Asn His Lys Lys
            130                 135                 140

        Gly Lys Asp Cys Ser Lys Leu Gly Lys Lys Cys Ile Tyr Gln Gln Leu
        145                 150                 155                 160

        Val Arg Gly Arg Lys Ile Arg Arg Ser Ser Glu Glu His Leu Arg Gln
                        165                 170                 175

        Thr Arg Ser Glu Thr Met Arg Asn Ser Val Lys Ser Ser Phe His Asp
                    180                 185                 190

        Pro Lys Leu Lys Gly Lys Pro Ser Arg Glu Arg Tyr Val Thr His Asn
                    195                 200                 205

        Arg Ala His Trp
            210
```

<210> 7
<211> 195
<212> PRT
<213> Homo sapiens

<400> 7

Ala Pro Glu Thr Ala Val Leu Gly Ala Glu Leu Ser Ala Val Gly Glu
1               5               10              15

Asn Gly Gly Glu Lys Pro Thr Pro Ser Pro Pro Trp Arg Leu Arg Arg
            20              25              30

Ser Lys Arg Cys Ser Cys Ser Ser Leu Met Asp Lys Glu Cys Val Tyr
        35              40              45

Phe Cys His Leu Asp Ile Ile Trp Val Asn Thr Pro Glu His Val Val
    50              55              60

Pro Tyr Gly Leu Gly Ser Pro Arg Ser Lys Arg Ala Leu Glu Asn Leu
65              70              75              80

Leu Pro Thr Lys Ala Thr Asp Arg Glu Asn Arg Cys Gln Cys Ala Ser
            85              90              95

Gln Lys Asp Lys Lys Cys Trp Asn Phe Cys Gln Ala Gly Lys Glu Leu
            100             105             110

Arg Ala Glu Asp Ile Met Glu Lys Asp Trp Asn Asn His Lys Lys Gly
        115             120             125

Lys Asp Cys Ser Lys Leu Gly Lys Lys Cys Ile Tyr Gln Gln Leu Val
    130             135             140

Arg Gly Arg Lys Ile Arg Arg Ser Ser Glu Glu His Leu Arg Gln Thr
145             150             155             160

Arg Ser Glu Thr Met Arg Asn Ser Val Lys Ser Ser Phe His Asp Pro
            165             170             175

Lys Leu Lys Gly Lys Pro Ser Arg Glu Arg Tyr Val Thr His Asn Arg
        180             185             190

Ala His Trp
        195

<210> 8
<211> 21
<212> PRT
<213> Homo sapiens

<400> 8

Cys Ser Cys Ser Ser Leu Met Asp Lys Glu Cys Val Tyr Phe Cys His
1               5               10              15

Leu Asp Ile Ile Trp
            20

<210> 9
<211> 45
<212> PRT

16

<213> Homo sapiens

<400> 9

```
Arg Ser Ser Glu Glu His Leu Arg Gln Thr Arg Ser Glu Thr Met Arg
1               5                   10                  15

Asn Ser Val Lys Ser Ser Phe His Asp Pro Lys Leu Lys Gly Lys Pro
                20              25              30

Ser Arg Glu Arg Tyr Val Thr His Asn Arg Ala His Trp
            35              40              45
```

<210> 10
<211> 38
<212> PRT
<213> Homo sapiens

<400> 10

```
Cys Ser Cys Ser Ser Leu Met Asp Lys Glu Cys Val Tyr Phe Cys His
1               5                   10                  15

Leu Asp Ile Ile Trp Val Asn Thr Pro Glu His Val Val Pro Tyr Gly
                20              25              30

Leu Gly Ser Pro Arg Ser
            35
```

<210> 11
<211> 164
<212> PRT
<213> Homo sapiens

<400> 11

```
Met Pro Asp Thr Met Leu Pro Ala Cys Phe Leu Gly Leu Leu Ala Phe
1               5                   10                  15

Ser Ser Ala Cys Tyr Phe Gln Asn Cys Pro Arg Gly Gly Lys Arg Ala
                20              25              30

Met Ser Asp Leu Glu Leu Arg Gln Cys Leu Pro Cys Gly Pro Gly Gly
            35              40              45

Lys Gly Arg Cys Phe Gly Pro Ser Ile Cys Cys Ala Asp Glu Leu Gly
        50              55              60

Cys Phe Val Gly Thr Ala Glu Ala Leu Arg Cys Gln Glu Glu Asn Tyr
65              70              75                  80

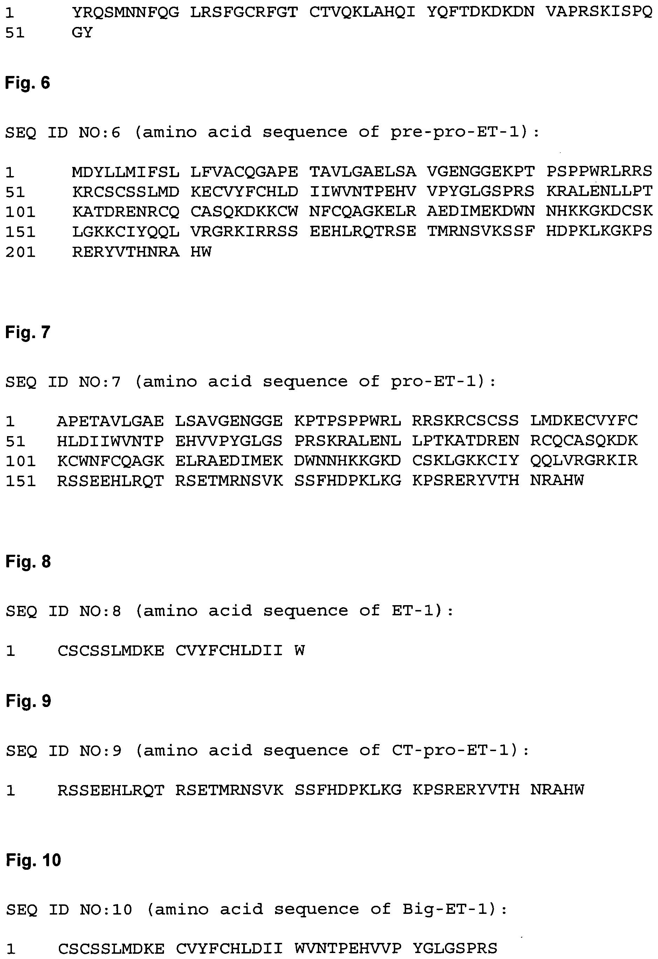

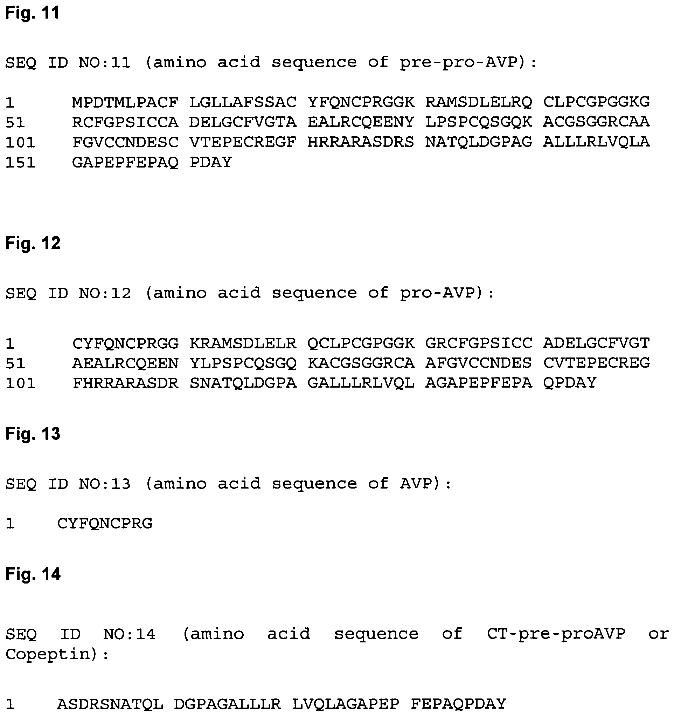

Leu Pro Ser Pro Cys Gln Ser Gly Gln Lys Ala Cys Gly Ser Gly Gly
                85              90                  95
```

Arg Cys Ala Ala Phe Gly Val Cys Cys Asn Asp Glu Ser Cys Val Thr
100 105 110

Glu Pro Glu Cys Arg Glu Gly Phe His Arg Arg Ala Arg Ala Ser Asp
115 120 125

Arg Ser Asn Ala Thr Gln Leu Asp Gly Pro Ala Gly Ala Leu Leu Leu
130 135 140

Arg Leu Val Gln Leu Ala Gly Ala Pro Glu Pro Phe Glu Pro Ala Gln
145 150 155 160

Pro Asp Ala Tyr

<210> 12
<211> 145
<212> PRT
<213> Homo sapiens

<400> 12

Cys Tyr Phe Gln Asn Cys Pro Arg Gly Gly Lys Arg Ala Met Ser Asp
1 5 10 15

Leu Glu Leu Arg Gln Cys Leu Pro Cys Gly Pro Gly Gly Lys Gly Arg
20 25 30

Cys Phe Gly Pro Ser Ile Cys Cys Ala Asp Glu Leu Gly Cys Phe Val
35 40 45

Gly Thr Ala Glu Ala Leu Arg Cys Gln Glu Glu Asn Tyr Leu Pro Ser
50 55 60

Pro Cys Gln Ser Gly Gln Lys Ala Cys Gly Ser Gly Gly Arg Cys Ala
65 70 75 80

Ala Phe Gly Val Cys Cys Asn Asp Glu Ser Cys Val Thr Glu Pro Glu
85 90 95

Cys Arg Glu Gly Phe His Arg Arg Ala Arg Ala Ser Asp Arg Ser Asn
100 105 110

Ala Thr Gln Leu Asp Gly Pro Ala Gly Ala Leu Leu Leu Arg Leu Val
115 120 125

Gln Leu Ala Gly Ala Pro Glu Pro Phe Glu Pro Ala Gln Pro Asp Ala
130 135 140

Tyr
145

<210> 13
<211> 9
<212> PRT

<213> Homo sapiens

<400> 13

Cys Tyr Phe Gln Asn Cys Pro Arg Gly
1               5

<210> 14
<211> 39
<212> PRT
<213> Homo sapiens

<400> 14

Ala Ser Asp Arg Ser Asn Ala Thr Gln Leu Asp Gly Pro Ala Gly Ala
1               5                   10                  15

Leu Leu Leu Arg Leu Val Gln Leu Ala Gly Ala Pro Glu Pro Phe Glu
            20              25                  30

Pro Ala Gln Pro Asp Ala Tyr
            35

**Claims**

1. An in vitro-method for classifying a foodstuff and/or beverage and/or diet and/or nutrition regimen and/or medicament in view of an effect on the cardiovascular system of a subject, comprising determining the postprandial relative level of one or more cardiovascular markers in said subject comprising:

   a. determining the basal level of one or more cardiovascular markers in said subject,
   b. determining the postprandial level of said one or more cardiovascular markers,
   c. calculating the relative level of one or more cardiovascular markers from the values obtained in steps a and b, wherein the relative level of markers is determined as follows:

$$X = 100 \times [\text{postprandial}]/[\text{basal}],$$

   and
   d. wherein a postprandial relative increase of the level of said one or more cardiovascular markers of more than 5%, preferably between more than 5 and up to 20% in said subject is correlated with a positive effect of said foodstuff and/or beverage and/or diet and/or nutrition regimen and/or medicament on the on the cardiovascular system of said subject, wherein a postprandial relative increase or decrease of the level of said one or more cardiovascular markers of about 5% in said subject is correlated to a neutral effect of said foodstuff and/or beverage and/or diet and/or nutrition regimen and/or medicament on the cardiovascular system of said subject, and wherein a postprandial relative decrease of the level of said one or more cardiovascular markers of more than 5% in said subject is correlated to a negative effect of said foodstuff and/or beverage and/or diet and/or nutrition regimen and/or medicament on the cardiovascular system of said subject,

   wherein one of the cardiovascular markers is MR-proANP, and
   wherein said relative level is determined with an assay having an intrassay precision of < 5% in the measuring range.

2. The in vitro-method according to claim 1, wherein the relative level of one or more cardiovascular markers is determined with an assay having a sensitivity 1 nmol/L or lower, preferably 100 pmol/L or lower, more preferably 10 pmol/L or lower, even more preferably 1 pmol/L or lower, most preferably 0.5 pmol/L or lower.

3. The in vitro-method according to claim 1 or 2, wherein the basal level of said cardiovascular marker and the postprandial level of said cardiovascular marker in said subject are determined with an immunoassay.

4. The use of an assay having an intraassay precision of < 5% in the measuring range for determining the postprandial change of the level of one or more cardiovascular markers of a subject relative to the basal level of said markers of said subject, wherein the assay is capable of detecting a decrease of the level of said one or more cardiovascular markers and capable of detecting an increase of the level of said one or more cardiovascular markers, wherein said change in the level of one or more cardiovascular markers is used to classify a foodstuff and/or beverage and/or diet and/or nutrition regimen and/or medicament in view of an effect on the cardiovascular system of a subject, wherein one of the cardiovascular markers is MR-proANP, wherein the change of the level of one or more markers is determined as follows:

$$X = 100 \times [postprandial]/[basal].$$

5. The use of an assay according to claim 4, wherein the change is an increase or a decrease, and wherein the assay has sensitivity of 1 nmol/L or lower.

6. The use of an assay according to any of claims 4 to 5, wherein the assay is an immunoassay.

7. The use of an assay according to any of claims 4 to 6 or the in vitro-method according to any of claims 1 to 3, wherein said change in the level of one or more cardiovascular markers is used to classify a diabetes medicament.

8. The use of an assay according to any of claims 4 to 7 or the in vitro-method according to any of claims 1 to 3 and 7, wherein said subject is a human being having a medical condition associated with the cardiovascular system and/or metabolic system

9. The use of an assay according to any of claims 4 to 8 or the in vitro-method according to any of claims 1 to 3 and 7 to 8, wherein said subject is a human being suffering from diabetes.

**Patentansprüche**

1. *In vitro-Verfahren* zum Klassifizieren eines Lebensmittels und/oder Getränkes und/oder Diät- und/oder Ernährungsregimes und/oder Medikaments im Hinblick auf eine Wirkung auf das kardiovaskuläre System eines Subjekts, umfassend den postprandialen relativen Wert eines oder mehrerer kardiovaskulären Marker in dem Subjekt, umfassend:

a. Bestimmen des Grundwerts eines oder mehrerer kardiovaskulärer Marker in dem Subjekt,
b. Bestimmen des postprandialen Wertes des einen oder der mehreren kardiovaskulären Marker,
c. Berechnen des relativen Werts eines oder mehrerer kardiovaskulären Marker aus den in den Schritten a und b erhaltenen Werten, wobei der relative Wert der Marker wie folgt bestimmt wird:

$$X = 100 \times [postprandial]/[basal]$$

und
d. wobei eine postprandiale relative Erhöhungg des Werts des einen oder der mehreren kardiovaskulären Marker von mehr als 5 %, vorzugsweise zwischen mehr als 5 und bis zu 20 %, bei dem Subjekt mit einer positiven Wirkung des Nahrungsmittels und/oder des Getränks und/oder des Diät- und/oder Ernährungsregimes und/oder Medikaments auf das kardiovaskuläre System des Subjekts korreliert ist, wobei eine postprandiale relative Erhöhung oder Abnahme des Werts des einen oder der mehreren kardiovaskulären Marker von etwa 5 % bei dem Subjekt mit einer neutralen Wirkung des Nahrungsmittels und/oder Getränkes und/oder Diät- und/oder Ernährungsregimes und/oder Medikaments auf das kardiovaskuläre System des Subjekts korreliert ist, und wobei eine postprandiale relative Abnahme des Werts des einen oder der mehreren kardiovaskulären Marker von mehr als 5 % in das Subjekt mit einer negativen Auswirkung des Nahrungsmittels und/oder des Getränks und/oder des Diät- und/oder Ernährungsregime und/oder des Medikaments auf das kardiovaskuläre System des Subjekts korreliert ist, wobei einer der kardiovaskulären Marker MR-proANP ist, und wobei der relative Wert mit einem Assay bestimmt wird, der eine Intrassay-Genauigkeit von <5 % im Messbereich hat.

**2.** *In vitro-Verfahren* nach Anspruch 1, wobei der relative Wert von einem oder mehreren kardiovaskulären Marker mit einem Assay mit einer Empfindlichkeit von 1 nmol/l oder weniger, vorzugsweise 100 pmol/l oder weniger, stärker bevorzugt 10 pmol/l oder weniger, noch bevorzugter 1 pmol/l oder weniger, am meisten bevorzugt 0,5 pmol/l oder weniger bestimmt wird.

**3.** *In vitro-Verfahren* nach Anspruch 1 oder 2, wobei der Grundwert des kardiovaskulären Markers und der postprandiale Wert des kardiovaskulären Markers in dem Subjekt mit einem Immunoassay bestimmt werden.

**4.** Verwendung eines Assays mit einer Intrassay-Genauigkeit von <5 % im Messbereich zur Bestimmung der postprandialen Änderung des Werts von einem oder mehreren kardiovaskulären Marker eines Subjekt relativ zu dem Grundwert der Marker des Subjektes, wobei der Assay in der Lage ist, eine Abnahme des Werts des einen oder der mehreren kardiovaskulären Marker zu detektieren und einen Anstieg des Werts des einen oder der mehreren kardiovaskulären Marker zu detektieren, wobei die Änderung des Werts eines oder mehrerer kardiovaskulärer Marker dazu verwendet wird ein Nahrungsmittel und/oder ein Getränk und/oder ein Diät- und/oder Ernährungsregime und/oder ein Medikament im Hinblick auf eine Wirkung auf das kardiovaskuläre System eines Subjekts zu klassifizieren, wobei einer der kardiovaskulären Marker MR-proANP ist, wobei die Änderung des Werts von einem oder mehreren Markern wie folgt bestimmt wird:

$$X = 100 \text{ x } [\text{postprandial}]/[\text{basal}].$$

**5.** Verwendung eines Assays nach Anspruch 4, wobei die Änderung eine Erhöhung oder eine Verringerung ist, und wobei der Assay eine Empfindlichkeit von 1 nmol/l oder niedriger hat.

**6.** Verwendung eines Assays nach einem der Ansprüche 4 bis 5, wobei der Assay ein Immunoassay ist.

**7.** Verwendung eines Assays nach einem der Ansprüche 4 bis 6 oder des *In vitro-*Verfahrens nach einem der Ansprüche 1 bis 3, wobei die Änderung des Werts von einer oder mehreren kardiovaskulären Marker verwendet wird, um ein Diabetes-Medikament zu klassifizieren.

**8.** Verwendung eines Assays nach einem der Ansprüche 4 bis 7 oder des *In vitro-*Verfahrens nach einem der Ansprüche 1 bis 3 und 7, wobei das Subjekt ein Mensch ist, der eine Erkrankung aufweist, welche dem kardiovaskulären System und/oder dem metabolischen System zugeordnet ist.

**9.** Verwendung eines Assays nach einem der Ansprüche 4 bis 8 oder des *In vitro-*Verfahrens nach einem der Ansprüche 1 bis 3 und 7 bis 8, wobei das Subjekt ein Mensch ist, der an Diabetes leidet.

**Revendications**

**1.** Procédé in vitro permettant la classification de denrées alimentaires et/ou de boissons et/ou de régimes alimentaires et/ou de traitements nutritionnels et/ou de médicaments concernant leur effet sur le système cardiovasculaire d'un sujet, consistant à déterminer le taux relatif postprandial d'un ou de plusieurs marqueurs cardiovasculaires chez ledit sujet comprenant :

a. la détermination du taux basal d'un ou de plusieurs marqueurs cardiovasculaires chez ledit sujet,
b. la détermination du taux postprandial dudit ou desdits marqueurs cardiovasculaires,
c. le calcul du taux relatif d'un ou de plusieurs marqueurs cardiovasculaires à partir des valeurs obtenues aux étapes a et b, le taux relatif de marqueurs étant déterminé comme suit :

$$X = 100 \text{ x } [\text{postprandial}]/[\text{basal}],$$

et

d. une augmentation relative postprandiale du taux dudit ou desdits marqueurs cardiovasculaires de plus de 5 %, de préférence compris entre plus de 5 % et jusqu'à 20 % chez ledit sujet, est corrélée à un effet positif desdits denrées alimentaires et/ou boissons et/ou régimes alimentaires et/ou traitements nutritionnels et/ou médicaments sur le système cardiovasculaire dudit sujet, une augmentation ou une diminution relative postprandiale du taux dudit ou desdits marqueurs cardiovasculaires d'environ 5 % chez ledit sujet étant corrélée à un effet neutre desdits denrées alimentaires et/ou boissons et/ou régimes alimentaires et/ou traitements nutritionnels et/ou médicaments sur le système cardiovasculaire dudit sujet, et une diminution relative postprandiale du taux dudit ou desdits marqueurs cardiovasculaires de plus de 5 % chez ledit sujet est corrélée à un effet négatif desdits denrées alimentaires et/ou boissons et/ou régimes alimentaires et/ou traitements nutritionnels et/ou médicaments sur le système cardiovasculaire dudit sujet, un des marqueurs cardiovasculaires étant MR-proANP, et ledit taux relatif étant déterminé par un dosage présentant une précision intra-analyse de < 5 % dans la plage de mesure.

2. Procédé in vitro selon la revendication 1, dans lequel le taux relatif d'un ou de plusieurs marqueurs cardiovasculaires est déterminé par un dosage ayant une sensibilité de 1 nmol/l ou inférieure, préférablement de 100 pmol/l ou inférieure, plus préférablement de 10 pmol/l ou moins, plus préférablement encore de 1 pmol/l ou moins, idéalement de 0,5 pmol/l ou moins.

3. Procédé in vitro selon la revendication 1 ou 2, dans lequel le taux basal dudit marqueur cardiovasculaire et le taux postprandial dudit marqueur cardiovasculaire chez ledit sujet sont déterminés par un dosage immunologique.

4. Utilisation d'un dosage comportant une précision intra-analyse de < 5 % dans la plage de mesure pour permettre la détermination de la variation postprandiale du taux d'un ou de plusieurs marqueurs cardiovasculaires d'un sujet par rapport au taux basal desdits marqueurs dudit sujet, le dosage étant capable de détecter une diminution du taux dudit ou desdits marqueurs cardiovasculaires et de détecter une augmentation du taux dudit ou desdits marqueurs cardiovasculaires, ladite variation du taux d'un ou de plusieurs marqueurs cardiovasculaires étant utilisée pour permettre la classification des denrées alimentaires et/ou boissons et/ou régimes alimentaires et/ou traitements nutritionnels et/ou médicaments concernant leur effet sur le système cardiovasculaire d'un sujet, l'un des marqueurs cardiovasculaires étant MR-proANP, la variabilité du taux d'un ou de plusieurs marqueurs étant déterminée comme suit :

$$X = 100 \times [postprandial]/[basal],$$

5. Utilisation d'un dosage selon la revendication 4, dans laquelle la variabilité est une augmentation ou une diminution, le dosage présentant une sensibilité de 1 nmol/l ou moins.

6. Utilisation d'un dosage selon l'une quelconque des revendications 4 à 5, dans laquelle le dosage est un dosage immunologique.

7. Utilisation d'un dosage selon l'une quelconque des revendications 4 à 6 ou procédé in vitro selon l'une quelconque des revendications 1 à 3, ladite variabilité du taux d'un ou de plusieurs marqueurs cardiovasculaires étant utilisée pour la classification d'un médicament antidiabétique.

8. Utilisation d'un dosage selon l'une quelconque des revendications 4 à 7 ou procédé in vitro selon l'une quelconque des revendications 1 à 3 et 7, ledit sujet étant un être humain présentant un trouble médical associé au système cardiovasculaire et/ou au système métabolique.

9. Utilisation d'un dosage selon l'une quelconque des revendications 4 à 8 ou procédé in vitro selon l'une quelconque des revendications 1 à 3 et 7 à 8, ledit sujet étant un être humain souffrant de diabète.

**Fig. 1**

SEQ ID NO:1 (amino acid sequence of pro-ANP):

```
1      NPMYNAVSNA DLMDFKNLLD HLEEKMPLED EVVPPQVLSE PNEEAGAALS
51     PLPEVPPWTG EVSPAQRDGG ALGRGPWDSS DRSALLKSKL RALLTAPRSL
101    RRSSCFGGRM DRIGAQSGLG CNSFRY
```

**Fig. 2**

SEQ ID NO:2 (amino acid sequence of pre-pro-ADM):

```
1      MKLVSVALMY LGSLAFLGAD TARLDVASEF RKKWNKWALS RGKRELRMSS
51     SYPTGLADVK AGPAQTLIRP QDMKGASRSP EDSSPDAARI RVKRYRQSMN
101    NFQGLRSFGC RFGTCTVQKL AHQIYQFTDK DKDNVAPRSK ISPQGYGRRR
151    RRSLPEAGPG RTLVSSKPQA HGAPAPPSGS APHFL
```

**Fig. 3**

SEQ ID NO:3 (amino acid sequence of pro-ADM):

```
1      ARLDVASEFR KKWNKWALSR GKRELRMSSS YPTGLADVKA GPAQTLIRPQ
51     DMKGASRSPE DSSPDAARIR VKRYRQSMNN FQGLRSFGCR FGTCTVQKLA
101    HQIYQFTDKD KDNVAPRSKI SPQGYGRRRR RSLPEAGPGR TLVSSKPQAH
151    GAPAPPSGSA PHFL
```

**Fig. 4**

SEQ ID NO:4 (amino acid sequence of MR-pro-ADM):

```
1      ELRMSSSYPT GLADVKAGPA QTLIRPQDMK GASRSPEDSS
```

**Fig. 5**

SEQ ID NO:5 (amino acid sequence of ADM):

```
1      YRQSMNNFQG  LRSFGCRFGT  CTVQKLAHQI  YQFTDKDKDN  VAPRSKISPQ
51     GY
```

## Fig. 6

SEQ ID NO:6 (amino acid sequence of pre-pro-ET-1):

```
1      MDYLLMIFSL  LFVACQGAPE  TAVLGAELSA  VGENGGEKPT  PSPPWRLRRS
51     KRCSCSSLMD  KECVYFCHLD  IIWVNTPEHV  VPYGLGSPRS  KRALENLLPT
101    KATDRENRCQ  CASQKDKKCW  NFCQAGKELR  AEDIMEKDWN  NHKKGKDCSK
151    LGKKCIYQQL  VRGRKIRRSS  EEHLRQTRSE  TMRNSVKSSF  HDPKLKGKPS
201    RERYVTHNRA  HW
```

## Fig. 7

SEQ ID NO:7 (amino acid sequence of pro-ET-1):

```
1      APETAVLGAE  LSAVGENGGE  KPTPSPPWRL  RRSKRCSCSS  LMDKECVYFC
51     HLDIIWVNTP  EHVVPYGLGS  PRSKRALENL  LPTKATDREN  RCQCASQKDK
101    KCWNFCQAGK  ELRAEDIMEK  DWNNHKKGKD  CSKLGKKCIY  QQLVRGRKIR
151    RSSEEHLRQT  RSETMRNSVK  SSFHDPKLKG  KPSRERYVTH  NRAHW
```

## Fig. 8

SEQ ID NO:8 (amino acid sequence of ET-1):

```
1      CSCSSLMDKE  CVYFCHLDII  W
```

## Fig. 9

SEQ ID NO:9 (amino acid sequence of CT-pro-ET-1):

```
1      RSSEEHLRQT  RSETMRNSVK  SSFHDPKLKG  KPSRERYVTH  NRAHW
```

## Fig. 10

SEQ ID NO:10 (amino acid sequence of Big-ET-1):

```
1      CSCSSLMDKE  CVYFCHLDII  WVNTPEHVVP  YGLGSPRS
```

**Fig. 11**

SEQ ID NO:11 (amino acid sequence of pre-pro-AVP):

```
1       MPDTMLPACF LGLLAFSSAC YFQNCPRGGK RAMSDLELRQ CLPCGPGGKG
51      RCFGPSICCA DELGCFVGTA EALRCQEENY LPSPCQSGQK ACGSGGRCAA
101     FGVCCNDESC VTEPECREGF HRRARASDRS NATQLDGPAG ALLLRLVQLA
151     GAPEPFEPAQ PDAY
```

**Fig. 12**

SEQ ID NO:12 (amino acid sequence of pro-AVP):

```
1       CYFQNCPRGG KRAMSDLELR QCLPCGPGGK GRCFGPSICC ADELGCFVGT
51      AEALRCQEEN YLPSPCQSGQ KACGSGGRCA AFGVCCNDES CVTEPECREG
101     FHRRARASDR SNATQLDGPA GALLLRLVQL AGAPEPFEPA QPDAY
```

**Fig. 13**

SEQ ID NO:13 (amino acid sequence of AVP):

```
1       CYFQNCPRG
```

**Fig. 14**

SEQ ID NO:14 (amino acid sequence of CT-pre-proAVP or Copeptin):

```
1       ASDRSNATQL DGPAGALLLR LVQLAGAPEP FEPAQPDAY
```

Fig. 15

**A**

Fig. 15

**B**

FIG. 15

FIG. 15

D

**Fig. 16**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 08168107 A

### Non-patent literature cited in the description

- **MESSERLI FH et al.** Obesity and essential hypertension. Hemodynamics, intravascular volume, sodium excretion, and plasma renin activity. *Arch Intern Med,* 1981, vol. 141, 81-5
- **STELFOX HT et al.** Hemodynamic monitoring in obese patients: the impact of body mass index on cardiac output and stroke volume. *Crit Care Med,* 2006, vol. 34, 1243-6
- **RUSKOAHO H.** Cardiac hormones as diagnostic tools in heart failure. *Endocr Rev,* 2003, vol. 24, 341-56
- **POTTER LR et al.** Natriuretic peptides, their receptors, and cyclic guanosine monophosphate-dependent signaling functions. *Endocr Rev,* 2006, vol. 27, 47-72
- **OLSEN MH et al.** N-terminal pro brain natriuretic peptide is inversely related to metabolic cardiovascular risk factors and the metabolic syndrome. *Hypertension,* 2005, vol. 46, 660-6
- **WANG TJ et al.** Plasma natriuretic peptide levels and the risk of cardiovascular events and death. *N Engl J Med,* 2004, vol. 350, 655-63
- **BIBBINS-DOMINGO K et al.** N-terminal fragment of the prohormone brain-type natriuretic peptide (NT-proBNP), cardiovascular events, and mortality in patients with stable coronary heart disease. *Jama,* 2007, vol. 297, 169-76
- **KISTORP C et al.** N-terminal pro-brain natriuretic peptide, C-reactive protein, and urinary albumin levels as predictors of mortality and cardiovascular events in older adults. *Jama,* 2005, vol. 293, 1609-16
- **HAEHLING et al.** Comparison of midregional pro-atrial natriuretic peptide with N-terminal pro-B-type natriuretic peptide in predicting survival in patients with chronic heart failure. *J Am Coll Cardiol,* 2007, vol. 50, 1973-80
- **WANG TJ et al.** Impact of obesity on plasma natriuretic peptide levels. *Circulation,* 2004, vol. 109, 594-600
- **DAS SR et al.** Impact of body mass and body composition on circulating levels of natriuretic peptides: results from the Dallas Heart Study. *Circulation,* 2005, vol. 112, 2163-8
- **OLSEN MH et al.** Cardiovascular risk prediction by N-terminal pro brain natriuretic peptide and high sensitivity C-reactive protein is affected by age and sex. *J Hypertens,* 2008, vol. 26, 26-34
- **WANG TJ et al.** Association of plasma natriuretic peptide levels with metabolic risk factors in ambulatory individuals. *Circulation,* 2007, vol. 115, 1345-53
- **APPEL LJ et al.** Effects of protein, monounsaturated fat, and carbohydrate intake on blood pressure and serum lipids: results of the OmniHeart randomized trial. *Jama,* 2005, vol. 294, 2455-64
- **HANEFELD M et al.** Postprandial plasma glucose is an independent risk factor for increased carotid intima-media thickness in non-diabetic individuals. *Atherosclerosis,* 1999, vol. 144, 229-35
- **HANEFELD M et al.** The challenge of the Metabolic Syndrome. *Horm Metab Res,* 2007, vol. 39, 625-6
- **SUZUKI K. et al.** *Clin. Exp. Hypertension,* 2008, vol. 30 (5), 309-314
- The Immunoassay Handbook. Elsevier LTD, May 2005
- **HULTSCHIG C et al.** *Curr Opin Chem Biol.,* February 2006, vol. 10 (1), 4-10
- **MORGENTHALER NG et al.** *ClinChem,* 2004, vol. 50, 234-6
- **GRUNDY et al.** *Circulation,* 2005, vol. 112, 2735-3752
- **DE LORGERIL M et al.** Mediterranean diet, traditional risk factors, and the rate of cardiovascular complications after myocardial infarction: final report of the Lyon Diet Heart Study. *Circulation,* 1999, vol. 99 (6), 779-85
- Report of the Expert Committee on the Diagnosis and Classification of Diabetes Mellitus. *Diabetes Care,* 2000, vol. 23 (1), 4-19
- **DEFRONZO RA et al.** Glucose clamp technique: a method for quantifying insulin secretion and resistance. *Am J Physiol,* 1979, vol. 237, E214-23
- **MATTHEWS DR et al.** Homeostasis model assessment: insulin resistance and beta-cell function from fasting plasma glucose and insulin concentrations in man. *Diabetologia,* 1985, vol. 28, 412-9

- **MORGENTHALER NG et al.** Immunoluminometric assay for the midregion of pro-atrial natriuretic peptide in human plasma. *Clin Chem,* 2004, vol. 50, 234-6
- *Circulation,* 2002, vol. 106, 3143-3421
- **MORGENTHALER et al.** *Clin Chem,* 2005, vol. 51 (10), 1823-1829

- **MORGENTHALER et al.** *Clin Chem,* 2004, vol. 50 (1), 234-236
- **MORGENTHALER et al.** *Clin Chem,* 2006, vol. 52 (1), 112-119
- **PAPASSOTIRIOU et al.** *Clin Chem,* 2006, vol. 52 (6), 1144-1151